(19) Europäisches Patentamt
European Patent Office
Office européen des brevets

(11) EP 0 702 079 B1

(12) **EUROPEAN PATENT SPECIFICATION**

(45) Date of publication and mention of the grant of the patent:
**19.03.2003 Bulletin 2003/12**

(51) Int Cl.[7]: **C11D 1/83**, A61K 7/50, C11D 1/86

(21) Application number: **95114383.3**

(22) Date of filing: **13.09.1995**

# (54) Low-irritant detergent compositions

Wenig reizendes Reinigungsmittel

Compositions détergentes peu irritantes

(84) Designated Contracting States:
**DE ES FR**

(30) Priority: **13.09.1994 JP 21856394**

(43) Date of publication of application:
**20.03.1996 Bulletin 1996/12**

(73) Proprietor: **Kao Corporation**
**Chuo-Ku Tokyo 103 (JP)**

(72) Inventors:
- **Kobayashi, Takatoshi, c/o Kao Corporation**
  **Haga-gun, Tochigi-ken (JP)**
- **Azuma, Toshikazu**
  **Wakayama-shi, Wakayama-ken (JP)**
- **Isobe, Kazuo**
  **Wakayama-shi, Wakayama-ken (JP)**
- **Pujol, Enrique**
  **E-08013 Barcelona (ES)**
- **Pujadas, Francisco**
  **E-08021 Barcelona (ES)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 007 120**
**EP-A- 0 586 323**
**FR-A- 2 390 498**
**GB-A- 1 333 475**
**US-A- 4 256 611**
**US-A- 4 343 726**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 702 079 B1

## Description

### BACKGROUND OF THE INVENTION

Field of the Invention

This invention relates to a detergent composition which is low-irritant to the skin and exhibits excellent foaming properties and high detergency and is particularly suited as a detergent for the body, such as a shampoo, a liquid body soap, and a facial cleanser.

Description of Related Art

Detergents used in shampoos, liquid body soaps, facial cleansers, etc. mainly comprise an anionic surface active agent exemplified by lauryl ether sulfates and a lathering accelerator (hereinafter sometimes called a booster).
These kinds of detergents today are required to have various characteristics as well as high detergency.
For example, they are required:

(1) to be low-irritant to the body, the skin, the eyes, etc.;
(2) to be degraded by microorganisms in a relatively short time for the environmental consideration;
(3) to be a clear and uniform solution at room temperature and to maintain the state for a relatively long period of time;
(4) to undergo no change of the above state even when stored in a low or high temperature (e.g., -5 to 50°C), or, if it becomes non-fluid in a low temperature, to be capable of returning to its original state on being left to stand at room temperature without any external means, such as stirring;
(5) to give the hair long-lasting moisture and an improved feel (sense of touch) such as smoothness, after washing; and
(6) to have a competitive price.

In general, anionic surface active agents having high detergency cause slight to medium irritation to the skin. Even those of low irritation act to remove sebum protecting the skin and hair and natural moisturizing factors because of their high detergency, resulting in drying or roughening of the skin or hair.
In recent years, many techniques have been proposed in order to reduce irritation to the skin and improve the feel after washing without impairing the high foaming properties of anionic surface active agents. For example, reduction of irritation by combined use of a low-irritant surface active agent, such as alkyl polyglycosides or sulfosuccinates (Japanese Patent Publication 3-7718), improvement of the feel after washing by addition of a cationic surface active agent, a water-soluble polymer, an oily component, e.g., lanolin, an amino acid, etc. (Japanese Patent Application Laid-Open 1-128914), improvement of moisture retention by addition of glycerin, glycols, etc., addition of a nonionic surface active agent, addition of an amphoteric surface active agents, such as fatty acid amide propylbetaine, etc. have so far been proposed. Additionally, it has been proposed to incorporate a cationic polymer for the purpose of improving the feel the detergent using an alkyl polyglycoside in combination.
Further, Japanese Patent Publication 58-57475 and Japanese Patent Application Laid-Open 4-122799 disclose that a combination of an alkylene oxide adduct of a glycerin partial ester with inexpensive fatty acids having 10 to 18 carbon atoms and a higher alkyl ether sulfate has remarkably reduced irritation to the eyes and skin.
US-A-4 256 611 discloses a liquid detergent system comprising a combination of an anionic surfactant and a nonionic surfactant in the form of ethoxylated partial glycerol esters of a higher, detergent grade fatty acid and optionally containing a foam stabilizing agent.
EP-0 586 323 A1 discloses detergent compositions comprising ethoxylated glycerine and a mixture of ethoxylated fatty acid glycerides, wherein the weight ratio of mono-, di-and tri-ester is 46-90:9-30:1-15, respectively.
However, these proposals, although producing an effect of reducing irritation, are not always satisfactory in detergency and foaming properties.

### SUMMARY OF THE INVENTION

It is therefore an object of the present invention to provide a detergent composition which is low-irritant and excellent in foaming properties and detergency, and gives satisfactory feel (sense of touch) after washing.
In the light of the above-mentioned circumstances, the inventors of the present invention conducted extensive investigations and found, as a result, that a detergent composition accomplishing the above object can be obtained by formulating an alkylene oxide adduct of a glycerin partial ester consisting essentially of a monoester compound of

glycerin, di-and triester compounds of glycerin, and an alkylene oxide adduct of glycerin at a specific ratio and a specific anionic surface active agent in a specific ratio, and thus completed the present invention.

The present invention also provides a detergent composition comprising component (A) and component (B)':

(A) a glycerin derivative mixture consisting essentially of 25 to 85 % by weight of a monoester compound of glycerin, 5 to 65 % by weight of di- and triester compounds of glycerin, and 10 to 70 % by weight of an alkylene oxide adduct of glycerin; and

(B)' at least one surface active agent selected from a monoalkylphosphoric acid, a polyoxyethylene alkyl ether phosphoric acid, a polyoxyethylene alkyl ether acetic acid, or an alkyl polyglycoside, or a salt thereof ;

the weight ratio of component (A) to component (B)', (A)/(B)', being (5 to 80)/ (95 to 20); and

the total concentration of component (A) and component (B)' being 5 to 60 % by weight based on the detergent composition.

The detergent composition according to the present invention is low-irritant to the skin, exhibits excellent foaming properties and high detergency, and gives satisfactory feel after washing and is particularly useful as a detergent composition for the body, such as a shampoo, a liquid body soap or a facial cleanser.

## DETAILED DESCRIPTION OF THE INVENTION

The detergent composition of the present invention is described below in detail.

Component (A) in the detergent composition of the present invention will be described below.

The term "glycerin derivative mixture" as used herein means a mixture consisting essentially of a monoester compound of glycerin, a diester compound of glycerin, a triester compound of glycerin, and an alkylene oxide adduct of glycerin. The monoester compound of glycerin include i) a compound in which one of the three OH groups in glycerin is esterified, ii) a compound in which one of the three OH groups in glycerin is esterified and one of the remainder OH groups is added with an alkylene oxide, and iii) a compound in which one of the three OH groups in glycerin is esterified and two remainder OH groups are added with an alkylene oxide. The diester compound of glycerin include i) a compound in which two of the three OH groups in glycerin are esterified, and ii) a compound in which two of the three OH groups in glycerin are esterified and one remainder OH group is added with an alkylene oxide. Hereinafter, the monoester compound of the glycerin, the diester compound of the glycerin, and the triester compound of the glycerin will be simply referred to as "monoester compound", "diester compound" and "triester compound", respectively. Also, the alkylene oxide adduct of glycerin will be simply referred to as "alkylene oxide adduct".

In component (A), from the viewpoint of assurance of sufficient foaming properties and detergency, the glycerin derivative mixture consists essentially of 25 to 85 % by weight the monoester compound, 5 to 65 % by weight of the di- and triester compounds, and 10 to 70 % by weight of the alkylene oxide adduct. If the content of the monoester compound is less than 25 % by weight, or if the content of the di- and triester compounds or the content of the alkylene oxide adduct exceeds the above each range, foam volume of the detergent composition is reduced. If the content of the alkylene oxide adduct is smaller than the above range, detergency of the detergent composition is reduced. It is preferred that the glycerin derivative mixture consists essentially of 35 to 65 % by weight of the monoester compound, 10 to 40 % by weight of the di- and triester compounds, and 25 to 65 % by weight of the alkylene oxide adduct. It is more preferred that the glycerin derivative mixture consists essentially of 25 to 65 % by weight of the monoester compound, 5 to 40 % by weight of the di- and triester compounds, and 10 to 55 % by weight of the alkylene oxide adduct. It is still preferred that the glycerin derivative mixture consists essentially of 40 to 55 % by weight of the monoester compound, 15 to 30 % by weight of the di- and triester compounds, and 30 to 45 % by weight of the alkylene oxide adduct.

The weight ratio of (the monoester compound)/(the di- and triester compounds)/(the alkylene oxide adduct) in the above-mentioned glycerin derivative mixture can be obtained by reversed phase liquid chromatography according to a solvent gradient method using, for example, a methanol/water solvent system.

The process for preparing the glycerin derivative mixture having the above-mentioned weight ratio is not particularly limited. Examples of the process include a process of adding a prescribed amount of an alkylene oxide to a mixture of a monoglyceride, a diglyceride and a triglyceride having a specific mixing weight ratio; a process comprising glycerolyzing a triglyceride and glycerin in the presence of an alkali catalyst simultaneously with or followed by addition of a prescribed amount of an alkylene oxide; and a process comprising esterifying a fatty acid and glycerin, followed by addition of a prescribed amount of an alkylene oxide. Alternatively, an alkylene oxide adduct of glycerin may be added to a partial ester of glycerin containing substantially no alkylene oxide adduct of glycerin at such a mixing ratio as to result in the above-recited weight ratio. Of these processes, the process starting with a triglyceride and glycerin or the process starting with a fatty acid and glycerin are preferred for the economical consideration.

In component (A), the alkylene oxide to be added to glycerin and a partial ester of glycerin preferably includes

those having 2 to 4 carbon atoms. From the standpoint of water-solubility, foaming properties, and ease in handling, ethylene oxide is most preferred. The number of moles of an alkylene oxide to be added per 1 mole of a glycerin skeleton is preferably 1.7 to 17.0 moles in average, still preferably 2.5 to 13.0 moles in average. If the number of moles of an alkylene oxide to be added is less than 1.7 moles, the mono-, di- and triester compounds tend to have poor water solubility. If it exceeds 17.0, the detergency and foaming power of the detergent composition of the present invention tend to be reduced. Accordingly, the number of moles of an alkylene oxide to be added is preferably in the above range. In component (A), the alkylene oxide added to the monoester compound, the diester compound and the alkylene oxide adduct may be the same or different.

In component (A), the acyl group in the monoester compound, the diester compound and the triester compound is preferably a saturated or unsaturated acyl group having 8 to 18 carbon atoms. From the standpoint of prevention of coloring due to oxidation, detergency, and foaming properties, a saturated acyl group having 8 to 18 carbon atoms is still preferred. From the standpoint of the cost an acyl group derived from coconut oil fatty acid is particularly preferred. The acyl group in the monoester compound, the diester compound and the triester compound may be the same or different.

Component (B)' in the detergent composition of the present invention will be described below.

In component (B)', the monoalkylphosphoric acid, polyoxyethylene alkyl ether phosphoric acid, polyoxyethylene alkyl ether acetic acid, alkyl polyglycoside, and salts thereof, are not particularly limited, and any of those generally used in conventional fragrant products and cosmetics may be employed.

More specifically, the monoalkylphosphoric acid or a salt thereof includes compounds represented by formula (1):

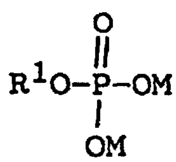

(1)

wherein $R^1$ represents a straight-chain or branched alkyl group having 8 to 18 carbon atoms, and M independently represents a hydrogen or a cation. Preferred examples of the monoalkylphosphoric acid and a salt thereof represented by formula (1) include laurylphosphoric acid and a salt thereof, myristyl-phosphoric acid and a salt thereof, cetylphosphoric acid and a salt thereof, and stearylphosphoric acid and a salt thereof.

The polyoxyethylene alkyl ether phosphoric acid or a salt thereof includes compounds represented by formula (2):

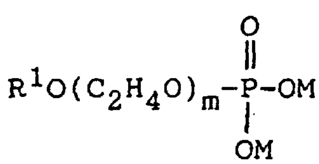

(2)

wherein $R^1$ and M are as defined above; and m represents a number of from 1 to 20.

Preferred examples of the polyoxyethylene alkyl ether phosphoric acid and a salt thereof represented by formula (2) include polyoxyethylene lauryl ether phosphoric acid and a salt thereof, polyoexyethylene myristyl ether phosphoric acid and a salt thereof, polyoxyethylene cetyl ether phosphoric acid and a salt thereof, and polyoxyethylene stearyl ether phosphoric acid and a salt thereof.

The polyoxyethylene alkyl ether acetic acid or a salt thereof includes compounds represented by formula (3):

$$R^1O(C_2H_4O)_nCH_2COOM \quad (3)$$

wherein $R^1$ and M are as defined above; and n represents a number of from 1 to 10.

Preferred examples of the polyoxyethylene alkyl ether acetic acid and a salt thereof represented by formula (3) include polyoxyethylene lauryl ether acetic acid and a salt thereof, polyoxyethylene cetyl ether acetic acid and a salt thereof, polyoxyethylene stearyl ether acetic acid and a salt thereof, and coconut oil derived alkyl ether acetic acid and a salt thereof.

The alkyl polyglycoside includes compounds represented by formula (10):

$$R^1(OR^4)_rG_s \qquad (10)$$

wherein $R^1$ is as defined above; $R^4$ represents an alkylene group having 2 to 4 carbon atoms; r represents a number of from 0 to 5; s represents a number of from 1 to 10; and G represents a reducing sugar having 5 or 6 carbon atoms.

Preferred examples of the alkyl polyglycoside represented by formula (10) include lauryl polyglycoside, myristyl polyglycoside, cetyl polyglycoside, and stearyl polyglycoside.

The salt as referred to above includes alkali metal salts, alkaline earth metal salts, ammonium salts, alkyl substituted ammonium salts, and alkanolamine salts, with sodium salts, potassium salts and triethanolamine salts being preferred.

Preferred of the above-described surface active agents are a monoalkylphosphoric acid, a polyoxyethylene alkyl ether acetic acid, an alkyl polyglycoside, and a salt thereof.

The component (A) and component (B)' are formulated such that the weight ratio of component (A) to component (B)', (A)/(B)', is (5 to 80)/(95 to 20), and the total concentration of component (A) and component (B)' is 5 to 60 by weight, based on the detergent composition of the present invention. If the weight ratio of component (A) is smaller than the above range, the detergent composition exhibits sufficient foaming properties but irritates the skin. If the weight ratio of component (A) is larger than the above range, the detergent composition is less irritant to the skin but has poor foaming properties and detergency particularly for use as a detergent for the body. The weight ratio, (A)/(B)', is preferably (20 to 60)/(80 to 40), still preferably (20 to 50)/(80 to 50).

The detergent composition of the present invention may further comprises a so-called booster as component (C) in order to enhance the foaming power. A booster which can be used for preference is at least one compound selected from an amphoteric surface active agent, a fatty acid alkanolamide, or an alkylamine oxide.

More specifically, examples of the amphoteric surface active agent include alkyldimethylaminoacetic acid betaines, such as lauryldimethylaminoacetic acid betaine and stearyldimethylaminoacetic acid betaine; alkylimidazolinium betaines, such as laurylcarboxymethylhydroxyethylimidazolinium betaine; and fatty acid amidopropylbetaines, such as lauramidopropyldimethylaminobetaine.

Examples of the fatty acid alkanolamide include coconut oil fatty acid diethanolamide, lauric diethanolamide, and lauric monoethanolamide ethylene oxide adduct.

The alkylamine oxide includes, for example, lauryldimethylamine oxide.

Preferred of these boosters are the amphoteric surface active agents and alkylamine oxides since the fatty acid alkanolamides are liable to cause turbidity. Further, use of the alkylamine oxide brings about enhanced foaming power but results in by-production, e.g., of nitrosoamine. Hence, (i) a fatty acid amidopropylbetaine and (ii) an alkylimidazolinium betaine having the formula shown below are especially suitable because they are free from the above-described disadvantages, have excellent effects of increasing foaming properties and of reducing skin irritation, and cause no appreciable increase in viscosity. The above-mentioned boosters may be used either individually or as a combination of two or more thereof.

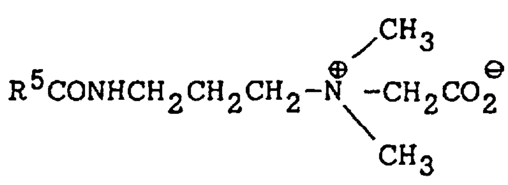

(i)

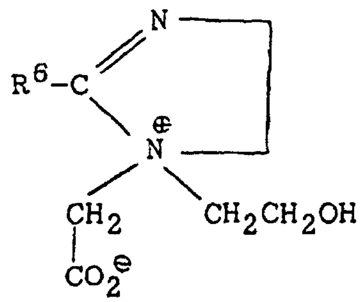

(ii)

wherein $R^5$ and $R^6$ independently represent an alkyl group having 7 to 17 carbon atoms.

Component (C), if used, is preferably added in an amount of 0.05 to 50% by weight based on the total amount of components (A) and (B). Addition of less than 0.05% by weight of component (C) tends to fail to produce any effect of enhancing foaming properties. If component (C) is added in an amount of more than 50% by weight, component (C) tends to be separated in the mixed system or cause turbidity. Therefore, the above-mentioned range is preferred.

The detergent composition according to the present invention may furthermore contain other components as far as the advantageous effects of the present invention are not impaired. Such components include, for example, oily components, such as higher alcohols, silicone oil, lanolin derivatives, protein derivatives, and polyethylene glycol fatty acid esters; water-soluble polymers, such as hydroxypropylmethyl cellulose and hydroxycellulose; cationic polymers, such as Polymer JR (produced by Union Carbide Corp.), Polycoat NH (produced by Henkels), Mercoat 550 (produced by Merck & Co., Inc.), and Gafcoat 755N (produced by GAF); polyhydric alcohols, such as sorbitol; chelating agents, such as ethylenediaminetetraacetic acid (EDTA); drugs, such as vitamins; animal or vegetable extracts or derivatives thereof, such as lecithin and gelatin; fine polymer particles, such as nylon and polyethylene; antiseptics, antimicrobial agents, pH adjusting agents, ultraviolet absorbers, coloring matter, perfumes, and the like.

The detergent composition of the present invention can be prepared by, for example, dissolving or dispersing in water components (A), (B) or (B)' and the above-mentioned optional components at the above-mentioned ratio, or components (A), (B) or (B)', (C) and the above-mentioned optional components at the above-mentioned ratio, in accordance with the conventional procedure.

The detergent composition of the present invention preferably has a form of liquid.

Examples

The present invention will now be illustrated in greater detail by way of Examples, but it should be understood that the present invention is not limited thereto.

In the following Preparation Example the composition of the glycerin derivative mixture was analyzed as follows.

Method of Composition Analysis:

Analysis was conducted with a high performance liquid chromatograph (Model L-6200, manufactured by Hitachi, Ltd.) using a separation column Wakosil-II 5C18 and a methanol/water mixture as a mobile phase. A differential refractive index detector (Model SE-51, manufactured by Showa Denko K.K.) was used for detection. The alkylene oxide adduct and the monoester compound were fractionated and, after solvent evaporation, weighed to calculate the respective weight ratios in the glycerin derivative mixture. The balance was taken as the amount of the diester compound and the triester compound.

PREPARATION EXAMPLE 1

Preparation of Glycerin Derivative Mixture

(1) Mixture (A-1):

Into a 3 ℓ pressure reactor were charged 481.1 g (2.28 mol) of coconut oil fatty acid, 384.5 g (4.18 mol) of glycerin, and, as a catalyst, 1.2 g of 85% potassium hydroxide. After displacing the inner atmosphere with nitrogen three times, the mixture was heated to 110°C in vacuo while stirring. The heating was continued up to 140°C and, after

confirming completion of the dehydration, 736.5 g (16.7 mol) of ethylene oxide was added thereto while maintaining the pressure inside the reactor at 2 to 3 $kg/cm^2$. After addition of all the ethylene oxide, the reaction mixture was allowed to react for 30 minutes and cooled, and the reaction product was taken out of the reactor to obtain Mixture (A-1).

Analysis by high performance liquid chromatography revealed that Mixture (A-1) consists essentially of 44% by weight of the monoester compound, 17% by weight of the total amount of the diester and triester compounds, and 39% by weight of the ethylene oxide adduct of glycerin.

(2) Mixture (A-2):

Into a 3 ℓ pressure reactor were charged 500 g (0.76 mol) of coconut oil fatty acid triglyceride, 174.8 g (1.90 mol) of glycerin, and, as a catalyst, 1.0 g of 85% potassium hydroxide. After displacing the inner atmosphere with nitrogen three times, the mixture was heated to 110°C in vacuo while stirring and allowed to react at that temperature for 1 hour. The heating was continued up to 140°C, and 836.9 g (19.0 mol) of ethylene oxide was added thereto while maintaining the pressure inside the reactor at 2 to 3 $kg/cm^2$. After addition of all the ethylene oxide, the reaction mixture was allowed to react for 30 minutes and cooled, and the reaction product was taken out of the reactor to obtain Mixture (A-2).

Analysis by high performance liquid chromatography revealed that Mixture (A-2) consists essentially of 43% by weight of the monoester compound, 18% by weight of the total amount of the diester and triester compounds, and 39% by weight of the ethylene oxide adduct of glycerin.

EXAMPLES 1 TO 4 (Reference Examples)

Shampoo compositions having the formulation shown in Table 1 were prepared using Mixtures (A-1) and (A-2) prepared in Preparation Example 1. The pH of the composition was adjusted to 6.5 to 7.0 with sodium hydroxide or citric acid. Each of the resulting shampoo compositions was organoleptically tested by 10 panel members, scored in 5 grades: 5 for "good", 4 for "slightly good", 3 for "moderate", 2 for "slightly bad", and 1 for "bad", and evaluated from the average of 10 scores based on the following standards. The results obtained are shown in Table 1.

Excellent ... Average score 4.0 or more
Good ... Average score 3.0 to 3.9
Fair ... Average score 2.0 to 2.9
Poor ... Average score 1.0 to 1.9

TABLE 1

| | | | Examples (Ref. Ex.) | | | |
|---|---|---|---|---|---|---|
| | | | 1 | 2 | 3 | 4 |
| Formulation (% by weight) | (A) | Mixture (A-1) | 10 | 5 | - | - |
| | | Mixture (A-2) | - | - | 15 | 8 |
| | (B) | Sodium lauryl-sulfate | 10 | - | - | 5 |
| | | Sodium lauryl ether sulfate | - | - | 5 | 5 |
| | | Sodium lauryl- sulfonate | 2 | 2 | 2 | 2 |
| | (C) | Lauric acid amide propylbetaine | 2 | 2 | 2 | 2 |
| | | Laurylimidazolinium betaine | 1 | 1 | 1 | 1 |
| | | Laurylamine oxide | 1 | 1 | 1 | 1 |
| | Cationic cellulose | | 0.2 | 0.2 | 0.2 | 0.2 |
| | Perfume | | 0.3 | 0.3 | 0.3 | 0.3 |
| | Water | | balance | balance | balance | balance |
| Appearance | | | clear | clear | clear | clear |

TABLE 1 (continued)

| | | Examples (Ref. Ex.) | | | |
|---|---|---|---|---|---|
| | | 1 | 2 | 3 | 4 |
| pH (1% solution) | | 6.5 | 6.8 | 6.4 | 6.6 |
| Viscosity (cp. 20°C) | | 2340 | 1650 | 2200 | 1530 |
| Foam Volume ($m\ell$) | | 330 | 335 | 325 | 330 |
| Evaluation | Lathering | Excellent | Good | Good | Excellent |
| | Feel | Excellent | Excellent | Good | Good |
| | Easy rinsing | Excellent | Excellent | Excellent | Excellent |

EXAMPLES 5 TO 9 (REFERENCE EXAMPLES) AND COMPARATIVE EXAMPLES 1 TO 4

Aqueous solutions of detergent compositions having the formulation shown in Table 2 were prepared and evaluated in terms of foam volume, percent detergency and skin irritation in accordance with the following test methods. The results obtained are shown in Table 2.

Measurement of Foam Volume:

Each composition and lanolin were dissolved in water having a hardness of 4° to prepare 100 ml of an aqueous solution containing 1% by weight of the total surface active agents and 0.5% by weight of lanolin. The aqueous solution was adjusted to pH 7 and heated to 40 °C. The aqueous solution was stirred for 5 minutes by reverse stirring. The volume of the foam after 30 seconds was measured.

Measurement of Percent Detergency:

Wool muslin of 10 cm x 10 cm was contaminated with a chloroform solution containing 5% by weight of a contaminant comprising 50% by weight of rape oil, 20% by weight of lauric acid, 10% by weight of oleic acid, 10% by weight of lanolin, 5% by weight of cholesterol, 3% by weight of paraffin, and 2% by weight of carbon black and dried. The thus contaminated cloth was washed by stirring in 100 ml of a 1% by weight aqueous solution of the detergent at 40°C for 10 minutes, rinsed well with tap water, and dried. The reflectance of the cloth was measured, and a percent detergency was obtained according to the following equation:

Percent Detergency (%) =

[(Reflectance after washing) - (Reflectance before

washing)]/[(Reflectance before contamination) -

(Reflectance before washing)] x 100

The higher the percent detergency, the higher the detergency.

Measurement of Skin Irritation:

A sample having the concentration adjusted to 6% by weight was applied to the skin of 5 subjects using a patch and maintained for 48 hours. After 24 hours from detaching of the patch, the skin reaction was judged based on the following criteria, and an average of the 5 subjects was taken as a result.

0 ... No change
1 ... Rash
2 ... Rash + edema
3 ... Rash + edema + small blisters
4 ... Tumorigenesis

TABLE 2

| | | Examples (Ref. Ex.) | | | | | Comparative Examples | | | |
|---|---|---|---|---|---|---|---|---|---|---|
| | | 5 | 6 | 7 | 8 | 9 | 1 | 2 | 3 | 4 |
| Composition of Component (A) | Origin of acyl group | coconut oil | coconut oil | coconut oil | coconut oil | palm oil | — | coconut oil | coconut oil | coconut oil |
| | The number of moles of EO added (per mole of glycerin skeleton) | 7.1 | 2.6 | 4.3 | 12.5 | 12.0 | — | 7.1 | 7.1 | 2.0 |
| | Monoester compound (% by weight) | 43 | 45 | 45 | 41 | 40 | — | 43 | 43 | 19 |
| | Di- and triester compounds (% by weight) | 18 | 18 | 20 | 12 | 10 | — | 18 | 18 | 76 |
| | EO-adduct of glycerin (% by weight) | 39 | 37 | 35 | 47 | 50 | — | 39 | 39 | 5 |
| Formulation (% by weight) | Component (A) | 4 | 7 | 10 | 13 | 16 | — | 3 | 17 | 10 |
| | Component (B): Sodium laurylsulfate | — | 8 | 5 | 4 | 1 | — | — | 3 | 5 |
| | Component (B): Sodium lauryl ether sulfate (2EO) | 16 | 5 | — | — | — | 20 | 17 | — | — |
| | Component (B): Sodium laurylsulfonate | — | — | 5 | 3 | 3 | — | — | — | 5 |
| | Ion-exchanged water | balance | balance | balance | balance | balance | balance | balance | balance | balance |
| Evaluation | Foam volume (mℓ) | 275 | 275 | 270 | 270 | 250 | 270 | 225 | 155 | 80 |
| | Percent detergency (%) | 82 | 90 | 93 | 92 | 81 | 40 | 55 | 43 | 18 |
| | Irritation score | 1.9 | 1.9 | 1.5 | 1.4 | 1.0 | 4.0 | 3.2 | 0.8 | 2.0 |

EXAMPLES 10 TO 18

Shampoo compositions having the formulation shown in Table 3 and liquid body soap compositions having

the formulation shown in Table 4 were prepared using Mixtures (A-1) and (A-2) prepared in Preparation Example 1 by the same manner as in Example 1. Each of the resulting shampoo compositions and liquid body soap compositions was evaluated by an organoleptic test in the same manner as in Example 1. The results obtained are shown in Tables 3 and 4.

TABLE 3

| | | | Examples | | | | |
|---|---|---|---|---|---|---|---|
| | | | 10 | 11 | 12* | 13 | 14 |
| Formulation (% by weight) | (A) | Mixture (A-1) | 8 | 8 | - | - | - |
| | | Mixture (A-2) | - | - | 8 | 8 | 8 |
| | | Sodium laurylphosphate | 4 | - | - | 6 | - |
| | | Sodium polyoxyethylene lauryl ether acetate (10E0) | - | 4 | - | - | 8 |
| | (B)' | Sodium polyoxyethylene laurylsulfosuccinate (10E0) | - | 8 | 6 | 6 | - |
| | | Sodium N-lauroyl-N-methyltaurine sodium salt | 8 | - | 6 | - | - |
| | | Lauryl polyglycoside (avg. degree of polymerization:1.5) | - | - | - | - | 4 |
| | (C) | Lauric acid amide propylbetaine | 4 | - | - | 2 | 4 |
| | | Laurylimidazolinium betaine | - | 4 | - | 2 | - |
| | | Laurylamine oxide | - | - | 4 | - | - |
| | Cationic. cellulose | | 0.2 | - | 0.2 | - | - |
| | Perfume | | 0.3 | 0.3 | 0.3 | 0.3 | 0.3 |
| | Water | | balance | balance | balance | balance | balance |
| Appearance | | | clear | clear | clear | clear | clear |
| pH (1% solution) | | | 6.7 | 8.5 | 6.5 | 6.6 | 6.8 |
| Foam Volume ($m\ell$) | | | 340 | 325 | 335 | 330 | 340 |
| Evaluation | Feel on shampooing | | Excellent | Good | Excellent | Good | Good |
| | Feel on rinsing | | Good | Excellent | Good | Excellent | Good |
| | Feel on drying with towel | | Excellent | Excellent | Excellent | Excellent | Excellent |
| | Feel after drying | | Excellent | Excellent | Excellent | Excellent | Excellent |

* Example 12 is a reference example

TABLE 4

| | | | Examples | | | |
|---|---|---|---|---|---|---|
| | | | 15 | 16 | 17 | 18 |
| Formulation (% by weight) | (A) | Mixture (A-1) | 6 | 6 | - | - |
| | | Mixture (A-2) | - | - | 6 | 6 |
| | (B)' | Potassium laurylphosphate | 8 | - | 8 | - |
| | | Sodium polyoxyethylene lauryl ether acetate (10E0) | - | 4 | - | 4 |
| | | Fatty acid triethanolamine salt | 6 | 10 | - | - |
| | | Sodium N-lauroyl-N-methyltaurine | - | - | 6 6 | 10 10 |
| | (C) | Lauric acid amide propylbetaine | 4 | - | - | 2 |
| | | Laurylimidazoliniun betaine | - | 4 | - | 2 |
| | | Laurylamine oxide | - | - | 4 | - |
| | Cationic cellulose | | - | - | 0.05 | 0.05 |
| | Perfume | | 0.3 | 0.3 | 0.3 | 0.3 |
| | Water | | balance | balance | balance | balance |
| Appearance | | | clear | clear | clear | clear |
| pH (1% solution) | | | 6.7 | 6.5 | 6.5 | 6.6 |
| Foam Volume ($m\ell$) | | | 340 | 325 | 335 | 330 |
| Evaluation | Feel on shampooing | | Good | Good | Excellent | Excellent |
| | Feel on rinsing | | Excellent | Excellent | Good | Excellent |
| | Feel on drying with towel | | Excellent | Excellent | Excellent | Good |
| | Feel after drying | | Excellent | Excellent | Excellent | Excellent |

EXAMPLES 19 TO 28 AND COMPARATIVE EXAMPLES 9 TO 12

Aqueous solutions of detergent compositions having the formulation shown in Table 5 were prepared and evaluated in terms of foam volume, percent detergency, skin irritation, and feel of the hands. The foam volume, percent detergency and skin irritation was evaluated by the same manner as in Example 5. The feel of the hands was evaluated in accordance with the following method. The results obtained are shown in Table 5.

Organoleptic Evaluation on Feel of Hands:

The detergent composition was used by 10 specialized panel members in washing their hands with tap water. After 30 minutes, the feel of the hands was scored in 5 grades: 5 for "moist and smooth", 4 for "slightly moist", 3 for "moderate", 2 for "slightly rough", and 1 for "very rough", and evaluated from the average grade of the 10 panel members based on the following standards.

Excellent ... Average score 4.0 or more
Good ... Average score 3.0 to 3.9
Fair ... Average score 2.0 to 2.9

Poor ... Average score 1.0 to 1.9

TABLE 5

| | | | Examples | | | | | | | | | | Comparative Examples | | | |
|---|---|---|---|---|---|---|---|---|---|---|---|---|---|---|---|---|
| | | | 19 | 20 | 21 | 22 | 23 | 24 | 25 | 26** | 27** | 28 | 9 | 10 | 11 | 12 |
| Composition of Component (A) | Origin of acyl group | | coconut oil | coconut oil | coconut oil | coconut oil | palm oil | coconut oil | coconut oil | coconut oil | coconut oil | coconut oil | — | coconut oil | coconut oil | coconut oil |
| | The number of moles of EO added (per mole of glycerin skeleton) | | 7.1 | 2.6 | 4.3 | 12.5 | 12.0 | 7.1 | 7.1 | 7.1 | 7.1 | 7.1 | — | 7.1 | 7.1 | 7.1 |
| | Monoester compound (% by weight) | | 43 | 45 | 45 | 41 | 40 | 43 | 43 | 43 | 43 | 43 | — | 43 | 43 | 19 |
| | Di- and triester compounds (% by weight) | | 18 | 18 | 20 | 12 | 10 | 18 | 18 | 18 | 18 | 18 | — | 18 | 18 | 76 |
| | Eo-adduct of glycerin (% by weight) | | 39 | 37 | 35 | 47 | 50 | 39 | 39 | 39 | 39 | 39 | — | 39 | 39 | 5 |
| Formulation (% by weight) | Component (A) | | 4 | 9 | 14 | 8 | 8 | 8 | 8 | 8 | 8 | 8 | — | 0.5 | 17 | 8 |
| | Component (B)' | *1 | 16 | 11 | 6 | — | — | 8 | 4 | — | — | — | 20 | — | 3 | — |
| | | *2 | — | — | — | — | — | — | — | — | — | — | — | 10.5 | — | — |
| | | *3 | — | — | — | 4 | — | — | — | — | — | — | — | — | — | 4 |
| | | *4 | — | — | — | 8 | 6 | — | 8 | — | — | — | — | — | — | 8 |
| | | *5 | — | — | — | — | 6 | — | — | 9 | — | — | — | — | — | — |
| | | *6 | — | — | — | — | — | — | — | — | 8 | — | — | — | — | — |
| | | *7 | — | — | — | — | — | — | — | — | 4 | — | — | — | — | — |
| | | *8 | — | — | — | — | — | — | — | 2 | — | — | — | — | — | — |
| | | *9 | — | — | — | — | — | — | — | — | — | 12 | — | — | — | — |
| | | *10 | — | — | — | — | — | 4 | — | 1 | — | — | — | — | — | — |
| | Ion-exchanged water | | balance | balance | balance | balance | balance | balance | balance | balance | balance | balance | balance | balance | balance | balance |
| Evaluation | Foam volume (ml) | | 270 | 270 | 265 | 270 | 260 | 275 | 280 | 295 | 265 | 275 | 270 | 270 | 155 | 80 |
| | Percent detergency (%) | | 92 | 90 | 93 | 92 | 89 | 90 | 92 | 95 | 89 | 88 | 54 | 62 | 34 | 18 |
| | Irritation score | | 1.3 | 0.9 | 0.7 | 1.0 | 1.0 | 0.9 | 0.8 | 0.7 | 1.0 | 0.8 | 1.5 | 1.5 | 0.8 | 1.2 |
| | Feel of hand skin | | Good | Excellent | Excellent | Excellent | Excellent | Good | Good | Excellent | Good | Excellent | Poor | Fair | Poor | Poor |

Note : *1 : Triethanolamine laurylphosphate
*2 : Sodium lauryl ether phosphate (2EO)
*3 : Sodium lauroylisetionate
*4 : Sodium polyoxyethylene (5) lauryl ether acetate
*5 : Sodium polyoxyethylene (4) lauric acid alkanolamide ether acetate
*6 : Sodium N-lauroyl-N-methylaminoethylsulfonate
*7 : Triethanolamine N-lauroylaminoethylcarboxylate
*8 : Sodium polyoxyethylene (3) laurylsulfosuccinate
*9 : Lauryl polyglycoside (average degree of polymerization: 1.5)
*10 : Triethanolamine myristic acid

** Examples 26 and 27 are reference examples

Many other variations and modifications of the invention will be apparent to those skilled in the art without departing from the spirit and scope of the invention. The above-described embodiments are, therefore, intended to be merely exemplary, and all such variations and modifications are intended to be included within the scope of the invention as defined in the appended claims.

## Claims

1. A detergent composition comprising component (A) and component (B)':

    (A) a glycerin derivative mixture consisting essentially of 25 to 85 % by weight of a monoester compound of glycerin, 5 to 65 % by weight of di- and triester compounds of glycerin, and 10 to 70 % by weight of an alkylene oxide adduct of glycerin; and
    (B)' at least one surface active agent selected from a monoalkylphosphoric acid, a polyoxyethylene alkyl ether phosphoric acid, a polyoxyethylene alkyl ether acetic acid, or an alkyl polyglycoside, or a salt thereof;

    the weight ratio of component (A) to component (B)', (A)/(B)', being (5 to 80)/ (95 to 20); and
    the total concentration of component (A) and component (B)' being 5 to 60 % by weight, based on the detergent composition.

2. The detergent composition according to claim 1, wherein, in component (B)', the alkyl group or acyl group in the surface active agent is a straight-chain or branched group having 8 to 18 carbon atoms.

3. The detergent composition according to claim 1, wherein, in component (B)', the salt in the surface active agent is an alkali metal, ammonium or alkanolamine salt.

4. The detergent composition according to claim 1, further comprising component (C):

    (C) at least one compound selected from an amphoteric surface active agent, a fatty acid alkanolamide or an alkylamine oxide in an amount of 0.05 to 50 % by weight, based on the total amount of components (A) and (B)'.

5. The detergent composition according to claim 1, wherein component (A) is obtainable by the process comprising adding a prescribed amount of an alkylene oxide to a mixture of a monoglyceride, a diglyceride and a triglyceride having a prescribed mixing weight ratio.

6. The detergent composition according to claim 1, wherein component (A) is obtainable by the process comprising glycerolyzing a triglyceride and glycerin in the presence of an alkali catalyst simultaneously with or followed by addition of a prescribed amount of an alkylene oxide.

7. The detergent composition according to claim 1, wherein component (A) is obtainable by the process comprising esterifying a fatty acid and glycerin, followed by addition of a prescribed amount of an alkylene oxide.

## Patentansprüche

1. Detergenszusammensetzung, umfassend eine Komponente (A) und eine Komponente (B)':

    (A) eine Glycerinderivatmischung, bestehend im wesentlichen aus 25 bis 85 Gew.% einer Monoesterverbindung von Glycerin, 5 bis 65 Gew.% von Dioder Triesterverbindungen von Glycerin und 10 bis 70 Gew.% eines Alkylenoxid-Adduktes von Glycerin; und

    (B)' mindestens eines Tensids, ausgewählt aus einer Monoalkylphosphorsäure, einer Polyoxyethylenalkyletherphosphorsäure, einer Polyoxyethylenalkyletheressigsäure oder einem Alkylpolyglycosid, oder einem Salz hiervon;

    wobei das Gewichtsverhältnis der Komponente (A) zur Komponente (B)', (A)/(B)', (5 bis 80)/(95 bis 20) beträgt; und die Gesamtkonzentration der Komponente (A) und der Komponente (B)' 5 bis 60 Gew.% in bezug auf die Detergenszusammensetzung beträgt.

2. Detergenszusammensetzung gemäss Anspruch 1, wobei, in Komponente (B)', die Alkylgruppe oder Acylgruppe in dem Tensid eine geradkettige oder verzweigte Gruppe mit 8 bis 18 Kohlenstoffatomen ist.

3. Detergenszusammensetzung gemäss Anspruch 1, wobei, in Komponente (B)', das Salz in dem Tensid ein Alkalimetall-, Ammonium- oder Alkanolaminsalz ist.

4. Detergenszusammensetzung gemäss Anspruch 1, weiterhin umfassend die Komponente (C):

(C) mindestens eine Verbindung, ausgewählt aus einem amphoteren Tensid, einem Fettsäurealkanolamid oder einem Alkylaminoxid in einer Menge von 0,05 bis 50 Gew.% in bezug auf die Gesamtmenge der Komponenten (A) und (B)'.

5. Detergenszusammensetzung gemäss Anspruch 1, wobei die Komponente (A) durch ein Verfahren erhältlich ist, das die Zugabe einer vorbeschriebenen Menge eines Alkylenoxids zu einer Mischung eines Monoglycerids, eines Diglycerids und eines Triglycerids mit einem vorbestimmten Mischverhältnis umfasst.

6. Detergenszusammensetzung gemäss Anspruch 1, wobei die Komponente (A) durch ein Verfahren erhältlich ist, das die Glycerolisierung eines Triglycerids und von Glycerin in Anwesenheit eines Alkali-Katalysators gleichzeitig mit oder gefolgt von der Zugabe einer vorbestimmten Menge eines Alkylenoxids umfasst.

7. Detergenszusammensetzung gemäss Anspruch 1, wobei die Komponente (A) durch ein Verfahren erhältlich ist, das die Veresterung einer Fettsäure und Glycerin, gefolgt von der Zugabe einer vorbeschriebenen Menge eines Alkylenoxids, umfasst.

## Revendications

1. Composition détergente comprenant un composant (A) et un composant (B)' :

(A) un mélange de dérivés de glycérine essentiellement constitué de 25 à 85 % en poids d'un composé monoester de glycérine, de 5 à 65 % en poids de composés di- et tri-ester de glycérine et de 10 à 70 % en poids d'un produit d'addition d'oxyde d'alkylène de glycérine ; et
(B)' au moins un tensioactif choisi parmi un acide monoalkylphosphorique, un polyoxyéthylènealkyléther d'acide phosphorique, un polyoxyéthylènealkyléther d'acide acétique ou un alkylpolyglycoside, ou un sel de celui-ci ;

le rapport en poids du composant (A) au composant (B)', (A)/(B)' étant de (5 à 80)/(95 à 20) ; et
la concentration totale du composant (A) et du composant (B)' étant de 5 à 60 % en poids, par rapport à la composition détergente.

2. Composition détergente selon la revendication 1, dans laquelle, dans le composant (B)', le groupe alkyle ou le groupe acyle dans le tensioactif est un groupe à chaîne droite ou ramifié comportant 8 à 18 atomes de carbone.

3. Composition détergente selon la revendication 1, dans laquelle, dans le composant (B)', le sel dans le tensioactif est un sel de métal alcalin, d'ammonium ou d'alcanolamine.

4. Composition détergente selon la revendication 1, comprenant en outre un composant (C) :

(C) au moins un composé choisi parmi un tensioactif amphotère, un alcanolamide d'acide gras ou un oxyde d'alkylamine, en une quantité de 0,05 à 50 % en poids, par rapport à la quantité totale des composants (A) et (B)'.

5. Composition détergente selon la revendication 1, dans laquelle le composant (A) peut être obtenu par le procédé comprenant l'addition d'une quantité prescrite d'un oxyde d'alkylène à un mélange d'un monoglycéride, d'un diglycéride et d'un triglycéride ayant un rapport en poids de mélange prescrit.

6. Composition détergente selon la revendication 1, dans laquelle le composant (A) peut être obtenu par le procédé comprenant une glycérolyse d'un triglycéride et de glycérine en présence d'un catalyseur alcalin simultanément

avec, ou suivie par, une addition d'une quantité prescrite d'un oxyde d'alkylène.

**7.** Composition détergente selon la revendication 1, dans laquelle le composant (A) peut être obtenu par le procédé comprenant une estérification d'un acide gras et de glycérine, suivie par une addition d'une quantité prescrite d'un oxyde d'alkylène.